# EUROPEAN PATENT APPLICATION

(11) **EP 2 535 085 A1**
(43) Date of publication of application: **19.12.2012**
(21) Application number: 11741996.0
(22) Date of filing: 28.01.2011
(51) Int. Cl.: A61N 5/06

(54) **PHOTOTHERAPY DEVICE**

(30) Priority: 12.02.2010 JP 2010028474; 12.03.2010 JP 2010055745
(71) Applicant: Panasonic Corporation, Kadoma-shi Osaka 571-8501 (JP)
(72) Inventor: II, Yoshiteru, Chuo-ku Osaka 540-6207 (JP); SHIMADA, Takuo, Chuo-ku Osaka 540-6207 (JP); MINEHISA, Jiro, Chuo-ku Osaka 540-6207 (JP); KAWASE, Yuki, Chuo-ku Osaka 540-6207 (JP)
(74) Representative: Schwabe - Sandmair - Marx
(86) International application number: PCT/JP2011/000479
(87) International publication number: WO 2011/099245

(57) **Abstract**

Disclosed is a phototherapy device which can irradiate an affected part, which is to be irradiated with light, with light selectively and intensively and can prevent eyes to undergo the direct irradiation with intense light emitted from a light source. Specifically disclosed is a phototherapy device comprising: a light guide member which has a contact surface that contacts with an affected part and an opposed surface that faces the contact surface and which serves as a light guide path; and a first light source which can emit light having the main wavelength that depends on the wavelength range of infrared ray to the light guide path. In the phototherapy device, a material constituting the light guide member has a refractive index identical to or higher than the refractive index of a biological tissue.

## Description

### Technical Field

The present invention relates to a phototherapy device and, more preferably, to a phototherapy device used for therapeutic treatment of an affected part of a patient with rheumatism.

### Background Art

Conventional phototherapy devices apply light onto affected parts of the body such as hands, wrists, feet, ankles or knees to alleviate intense pain due to chronic non-infectious inflammation in muscles or joints or to treat rheumatoid arthritis. These conventional phototherapy devices are so configured as to apply therapeutic light from a light source onto an affected part in a non-contact manner either directly or via a light guide path (see, e.g., PTL 1).

### Citation List

### Patent Literature

PTL 1: Japanese Patent Application Laid-Open No.63-21069.

### Summary of Invention

### Technical Problem

The first disadvantage associated with the conventional phototherapy devices, however, is that therapeutic light cannot be selectively and intensively applied onto an affected part to be treated. More specifically, the conventional phototherapy devices are so configured as to apply therapeutic light even to body areas where therapeutic treatment is not needed, requiring a large amount of unwanted light that does not contribute to therapy. Accordingly, efficient therapy cannot be achieved with the conventional phototherapy devices. The conventional phototherapy devices thus require light that is intense enough to provide sufficient therapeutic efficacy to affected parts while considering light that is applied onto areas other than the affected part. For the reasons mentioned above, the conventional phototherapy devices require larger device size or, where upsizing is not intended, a device configuration that allows for irradiation onto a small specific site.

The second disadvantage associated with the conventional phototherapy devices is that there is a risk that intense light from a light source directly enters the eye of a patient. More specifically, the light source used for the phototherapy device is configured to emit intense light for enhanced phototherapeutic efficacy, and hence it is undesirable that intense light directly enters the eye of a patient. To cope with this problem, it is conceivable that a protection cover or like member is provided to prevent direct entry of intense light into the eye. However, there is a possibility that mishandling of such a protection cover results in direct entry of intense light to the eye. Such mishandling includes improper installment and missing due to forgetfulness.

The present invention has been accomplished in order to overcome at least one of the above-described disadvantages. An object of the present invention is therefore to provide a Phototherapy device that is capable of selective and intensive irradiation onto an affected part to be treated, can prevent direct entry of intense light from a light source into the eye of a patient, and is particularly useful for therapeutic treatment of a patient with rheumatism.

### Solution to Problem

To overcome at least one of the above-described disadvantages, a phototherapy device according to an embodiment of the present invention includes:
1) a light guide member having a contact surface to be contacted with an affected part, and an opposite surface opposing the contact surface, the light guide member serving as a light guide path; and
2) a first light source for applying light having a main wavelength in the wavelength range of near-infrared light into the light guide member,
   wherein a material constituting the light guide member has a refractive index equal to or higher than a refractive index of a biological tissue.
   With this configuration, the present invention attains the intended purposes.

### Advantageous Effects of Invention

A phototherapy device according to an embodiment of the present invention can provide effective therapy by selectively and intensively applying light onto an affected part to be treated. More specifically, when an affected part (e.g., hand) is brought into contact with the contact surface of the light guide member of the phototherapy device, intense light is emitted only from the contacted part of the light guide member for irradiation onto the affected part. With this configuration, the limited light source in the phototherapy device can be efficiently utilized for phototherapy.

The conventional phototherapy devices require larger device size for radiation of high-power light or, where upsizing is not intended, need to reduce the irradiation area. In contrast, the phototherapy device according to an embodiment of the present invention reduces need for increased device size and reduced irradiation area. Moreover, the phototherapy device overcomes the need to apply light onto areas other than the affected part. This leads to downsizing, increased therapeutic efficiency, and improved energy efficiency of the phototherapy device.

Furthermore, with the Phototherapy device according to an embodiment of the present invention, when an affected part (e.g., hand) contacts the contact surface of the light guide member, intense light is emitted only from the contacted part of the light guide member for irradiation onto the affected part. It is thus possible to reduce light emission from a non-contacted part on the contact surface of the light guide member, preventing possible direct entry of intense light to the eye of a patient.

The phototherapy device according to an embodiment of the present invention also allows intense light from a light source to be selectively applied onto an affected part that is in contact with the contact surface of the light guide member; therefore, there is no need to adjust the irradiation area according to the shape and size of the affected part being treated, nor to reset the same following the movement of the affected part. This also makes the device configuration simple.

### Brief Description of Drawings

FIG.1 is a perspective view illustrating a phototherapy device according to Embodiment 1 of the present invention;
FIG.2 is a perspective view illustrating a use state of the phototherapy device during use according to Embodiment 1 of the present invention;
FIG.3 is a front perspective view of the phototherapy device according to Embodiment 1 of the present invention;
FIG.4 is a lateral perspective view of the phototherapy device according to Embodiment 1 of the present invention;
FIG.5 is a cross-sectional view of a light guide member of the phototherapy device according to Embodiment 1 of the present invention;
FIG.6 is a bottom view of the light guide member of the phototherapy device according to Embodiment 1 of the present invention;
FIG.7 is a control block diagram of the phototherapy device according to Embodiment 1 of the present invention;
FIG.8 is an operation flow chart of the phototherapy device according to Embodiment 1 of the present invention;
FIG.9 is a cross-sectional view illustrating an irradiation from the light guide member of the phototherapy device according to Embodiment 1 of the present invention;
Fig.10 is a cross-sectional view illustrating an irradiation at the time when an affected part (biological tissue) is in contact with the light guide member of the phototherapy device according to Embodiment 1 of the present invention;
FIG.11 is a diagram for supplementing the description of intersection of main optical axes according to Embodiment 2 of the present invention;
FIG.12 illustrates main optical axes of light entered into a light guide member from a plurality of light sources of a phototherapy device according to Embodiment 2 of the present invention;
FIG.13 illustrates a state where a hand is placed on the light guide member of the phototherapy device according to Embodiment 2 of the present invention;
FIG.14 is a perspective view of a light guide member of a phototherapy device according to Embodiment 3 of the present invention;
FIG.15 is a front perspective view of a phototherapy device according to Embodiment 4 of the present invention;
FIG.16 illustrates a light guide member of the phototherapy device according to Embodiment 4 of the present invention;
FIG.17 is a cross-sectional view illustrating an irradiation into the light guide member of the phototherapy device according to Embodiment 4 of the present invention;
FIG.18 illustrates a light guide member of a phototherapy device according to Embodiment 5 of the present invention;
FIG.19 illustrates an end part of the light guide member of the phototherapy device according to Embodiment 5 of the present invention; and
FIG.20 illustrates another example of an end part of the light guide member of the phototherapy device according to Embodiment 5 of the present invention.

### Description of Embodiments

A phototherapy device according to an embodiment of the present invention includes a light guide member that serves as a light guide path, and a first light source for applying light into the light guide member.

The light guide member of the phototherapy device constitutes a light guide path by itself. The shape of the light guide member is not limited in particular; it may have a sheet shape, hollow semi-spherical shape or rod shape. The "spherical" as used in "semi-spherical shape" needs not to mean a perfect spherical shape, and includes oval spherical shape and other shapes.

The light guide member of the phototherapy device has a contact surface to which an affected part can be contacted. The affected part needs not to be brought into contact with the entire contact surface; the affected part needs only to be brought into contact with any desired part of the contact surface. A partial area of the contact surface may be pre-set as an area on which an affected part is to be placed (affected part zone). As will be described later, the therapeutic efficacy can be enhanced by concentrating light from a light source on the affected part zone.

The light guide member of the phototherapy device preferably has an opposite surface opposing the contact surface. The opposite surface needs to oppose the contact surface. For example, when the light guide member has a sheet shape and when one of the surfaces of the sheet is the contact surface, the other surface of the sheet is the opposite surface. Further, when the light guide member has a hollow semi-spherical shape and when the outer surface of the hollow semi-spherical body is the contact surface, the inner surface of the hollow semi-spherical body is the opposite surface.

A reflection member is preferably provided on the opposite surface. The reflection member refers to a member which reflects, to the contact surface side, light that has propagated through the light guide path configured by the light guide member and reached the opposite surface. The reflection member is, for example, a reflection film.

It is necessary that the material of the light guide member be transparent to the light propagating through the light guide path. It is also necessary that the material of the light guide member have a refractive index equal to or higher than a refractive index of a biological tissue.

First, the light propagating through the light guide path is light having a main wavelength within the near-infrared wavelength range, as will be described later.

Further, the refractive index of the biological tissue means a refractive index of a material constituting an affected part of a patient, that is, typically means a refractive index of a protein. The refractive indices of proteins range from about 1.4 to about 1.5. Therefore, it is preferable that the refractive index of the material of the light guide member be 1.4 or more. Examples of materials having a refractive index of 1.4 or more and being transparent to the near-infrared light include acrylic resins (refractive index: 1.49), polyethylene terephthalate resins (refractive index: 1.66), glass (refractive index: 1.51), and silicone rubbers having a refractive index adjusted to 1.4 or more.

The light guide member has a light guide path end surface having a light incident port through which light from the first light source enters. That is, the first light source is provided near the light guide path end surface. One or more first light sources may be provided, but it is preferable that more than one first light source is provided. When more than one first light source is provided, it is preferable that more than one light incident is provided correspondingly.

The first light source emits light having a main wavelength in a near-infrared wavelength range. The term " light having the main wavelength in the near-infrared wavelength range" means light which has a maximum peak in the near-infrared wavelength range in the wavelength distribution. The term "'near-infrared wavelength range" means a wavelength band of 750 nm to 2 µm.

Near-infrared light has high penetration into a biological tissue. There is a reality that phototherapy of a rheumatism-affected part provides sufficient therapeutic efficacy only when therapeutic light reaches the deep part of the tissue of the affected part (e.g., joints of fingers, hands or feet). For this reason, it is required that the first light source emit light which have a main wavelength in the near-infrared wavelength range.

On the other hand, the radiation used in phototherapy devices intended for thermotherapy to alleviate "pain" caused by chronic non-infectious inflammation of muscles and joints is generally mid- and far-infrared light having a wavelength of 3 µm or longer. However, mid- and far-infrared light is easily absorbed in water and therefore in interstitial fluid between cells of the body surface. Therefore, mid- and far-infrared light is less likely to reach the deep part of tissue and hence is not suitable for phototherapy of rheumatism-affected parts. When the light guide member is made of general-purpose material (e.g., acrylic resin or PET resin), mid- and far-infrared light is absorbed by the light guide member and hence cannot propagate therein. Thus, there also arises a problem that general-purpose materials cannot be employed as the material of the light guide member and therefore special material must be employed.

Visible light such as red light is absorbed by hemoglobin in blood and is therefore less likely to reach the deep part of tissue, rendering it unsuitable for phototherapy of rheumatism-affected parts.

For the reasons mentioned above, the light source used in the phototherapy device according to an embodiment of the present invention needs to emit near-infrared light. Especially, near infrared light having a wavelength in the range of 700 nm to 900 nm is particularly suitable because it is hard to be absorbed by water and hemoglobin, and can be used for a general-purpose light guide member.

As described above, it is preferable to provide more than one first light source. It is preferable that the first light sources are respectively provided such that the main optical axis of a first of the first light source and the main optical axis of a second of the first light source intersect each other in the light guide path. The light density at where the main optical axes intersect each other increases, masking it is possible to enhance the phototherapeutic efficacy by placing an affected part at the intersection. Therefore, phototherapeutic efficacy can be enhanced by providing a large number of intersections of the main optical axes in the affected part zone predetermined on the contact surface of the light guide member, so as to increase the density of intersections of the main optical axes in the affected part zone. The term "the density of intersections of the main optical axes" means the number of points at each of which two main optical axes intersect each other in a predetermined area. The details of the intersection of main optical axes will be described later with reference to the accompanying drawings.

It is preferable that the phototherapy device of the present invention include a base on which the light guide member is arranged. It is also preferable that the phototherapy device of the present invention include a lid which can cover the base. The lid can be freely opened and closed, and the light from the light source is prevented from directly entering the eye of a patient.

The disorder to receive phototherapy using the phototherapy device according to an embodiment of the present invention is not limited in particular; it is, for example, rheumatism. Rheumatism is a disorder that results from abnormal proliferation of synovial cells due to inflammation of the synovial membrane of a joint, destroying other tissues to induce swelling and deformation of the joint. In particular, rheumatism is a disorder in which severe symptoms usually appear in a joint of a hand, a foot or a finger, and in which, as the symptoms of the disease become severer, it becomes more difficult to move the joint of the hand, foot or finger.

Now, embodiments of a phototherapy device of the present invention will be described in detail with reference to the accompanying drawings.

### (Embodiment 1)

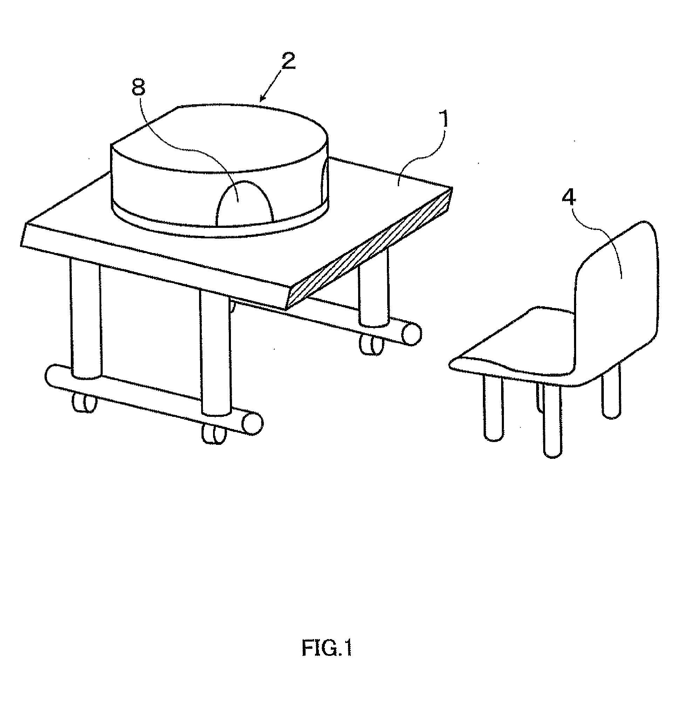

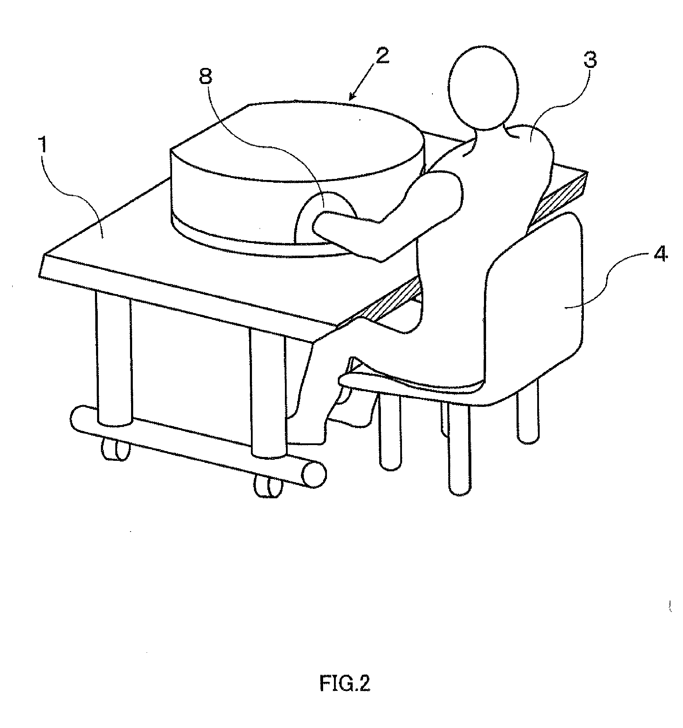

FIG.1 is a perspective view of a phototherapy device according to Embodiment 1 of the present invention. Phototherapy device 2 is mounted on desk 1. As shown in FIG.2, patient 3 can sit on chair 4 and receive phototherapy in his/ her house or in a hospital.

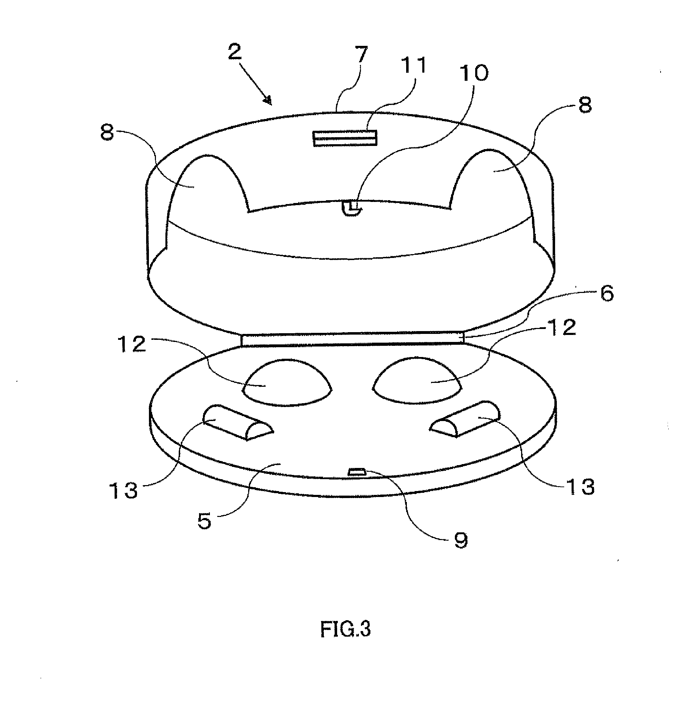

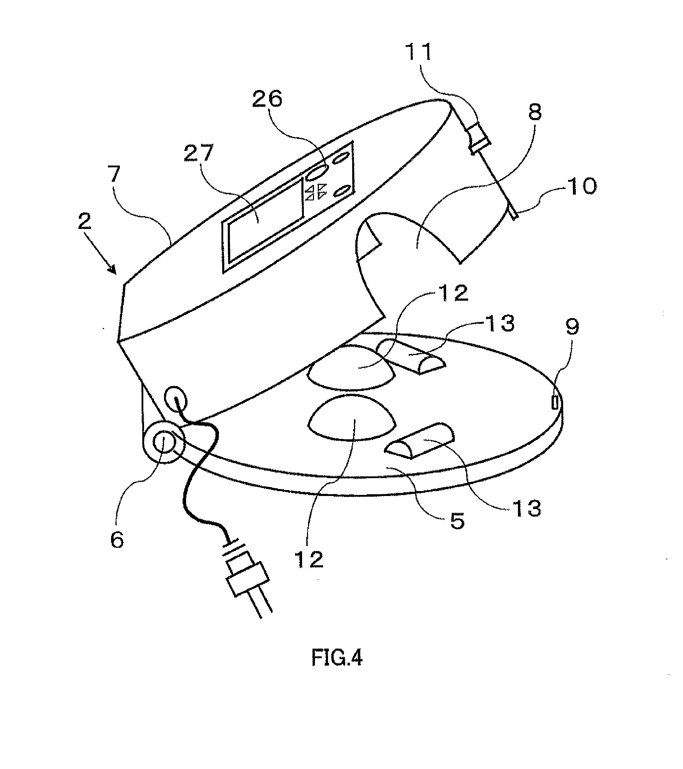

As shown in FIG.3 and FIG.4, phototherapy device 2 includes disc-like base 5 and lid 7. Lid 7 has a capped cylindrical shape whose lower surface is open. Lid 7 is connected to base 5 by hinge 6, and can cover the surface of base 5 in an openable and closable manner. When the surface of base 5 is covered by lid 7, intense light for use in phototherapy can be prevented from directly entering the eye of the patient.

Left and right two insertion ports 8 of affected parts are provided on the front surface side of the outer peripheral surface of lid 7, that is, on the side opposite to hinge 6. Further, hook 10 for locking lid 7 by being engaged with engagement hole 9 of base 5 is provided on the front surface side of the outer peripheral surface of lid 7 and between two insertion ports 8 of affected parts. Hook button 11 for releasing the lock is arranged above hook 10.

On the surface of base 5, left and right two light guide members 12 are provided at a predetermined spacing. Further, two mounting bodies 13 are provided between left and right insertion ports 8 and two light guide members 12.

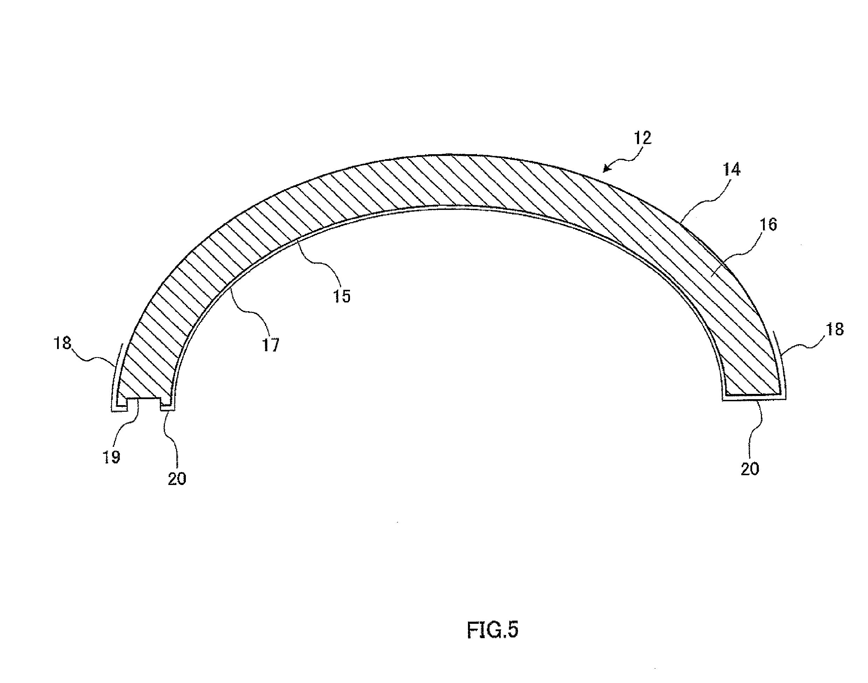

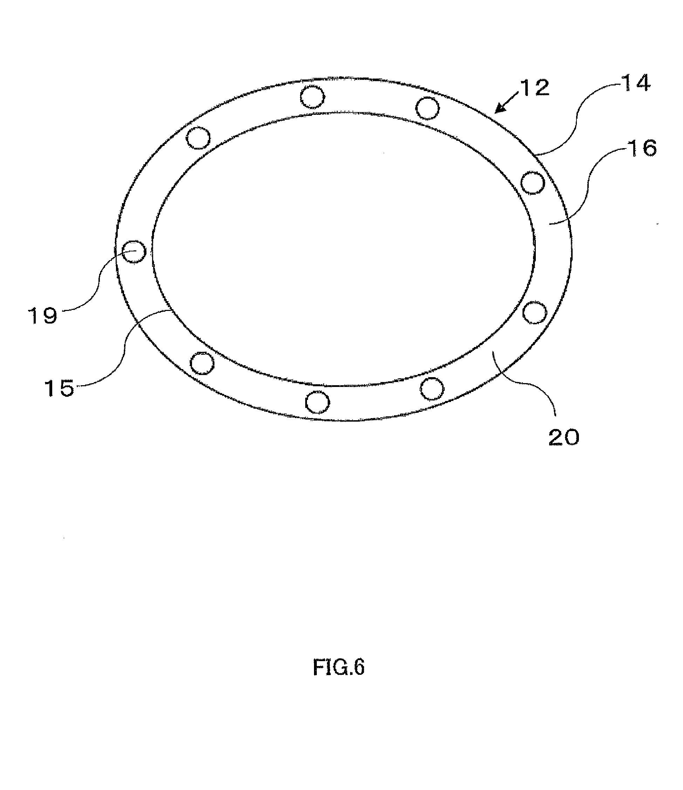

FIG.5 is a cross-sectional view of light guide member 12. FIG.6 is a bottom view of light guide member 12. As can be seen from FIG.5 and FIG.6, light guide member 12 forms a hollow semi-sphere. Each of the outer and inner surfaces of light guide member 12 is generally formed in a substantially semi-spherical shape. The outer surface of light guide member 12 is contact surface 14 which is brought into contact with an affected part (e.g., hand). The inner surface of light guide member 12 is opposite surface 15. In light guide member 12, a space between contact surface 14 (or outer surface) and opposite surface 15 (or inner surface) serves as light guide path 16. The thickness (distance between contact surface 14 and opposite surface 15) of light guide path 16 is substantially uniform.

The size of contact surface 14 of light guide member 12 is large enough to allow a hand to be placed thereon. Contact surface 14 of light guide member 12 is formed in a substantially semi-spherical shape. However, as can be seen from FIG.6, contact surface 14 is not formed in an exactly semi-spherical shape, but is formed such that one diameter (lateral diameter in the drawing) is slightly larger than the other diameter (vertical diameter in the drawing) perpendicular to that diameter. The contact surface of light guide member 12 is formed in a substantially semi-spherical shape in which the diameter in the direction perpendicular to the insertion direction from insertion port 8 is set slightly larger than the diameter in the insertion direction from insertion port 8. With this configuration, the hand can be easily put on contact surface 14.

Patients who have limited hand movement capabilities suck as those with rheumatism may find it difficult to properly place their hand on contact surface 14 of light guide member 12. Therefore, it is required that the size and shape of contact surface 14 of light guide member 12 be more suited to the size and shape of the hand. With this configuration, even patients with severe rheumatism can receive phototherapy by bringing their affected part into contact with the contact surface of light guide member 12 without requiring intense movement of the joints of the hand, foot or fingers, leaving them in a comfortable posture.

It is also preferable that reflection member 17, which reflects light to the contact surface 14 side, is provided on the entire surface of opposite surface 15 of light guide member 12. Furthermore, it is preferable that reflection member 18, which reflects light to the opposite surface 15 side, is provided in a lower part of the spherical surface of contact surface 14 of light guide member 12. Reflection members 17,18 are, for example, reflection films.

As shown in FIG.5, light incident port 19 is provided on the lower end surface of light guide path 16 of light guide member 12. It is preferable that more than one light incident ports 19 be provided as shown in FIG.6. Light from a light source (not shown) enters light guide path 16 through light incident port 19.

It is preferable that reflection member 20, which reflects light to the light guide path 16 side, is provided in the area other than the part of light incident port 19 on the lower end surface of light guide path 16. That is, light incident port 19 is a window at which reflection member 20 is not provided. Reflection member 20 is, for example, a reflection film.

The material of light guide member 12 is not limited in particular as long as the material is permeable to the light from the light source and has a refractive index equal to or higher than a refractive index of a biological tissue. The refractive index of the biological tissue is generally in the range of 1.4 to 1.5, and hence the refractive index of the material of light guide member 12 needs only to be 1.4 or more.

Specific examples of the material of light guide member 12 include, as described above, transparent acrylic resins, transparent polyethylene terephthalate (PET) resins, transparent glass, transparent silicone rubbers whose refractive index is adjusted to 1.4 or more. The silicone rubber whose refractive index is adjusted to 1.4 or more is a material having a refractive index higher than the refractive index of the air, and can thus be said that it is the material that has a refractive index comparable to that of a biological tissue.

By way of example, an embodiment where transparent acrylic resin is used as the material of light guide member 12 will now be discussed. The critical angle of light emitting to air from contact surface 14 of light guide member 12 is 42.155 degrees. On the other hand, the critical angle of light emitting from contact surface 14 of light guide member 12 to an affected part (e.g., a hand) in contact with contact surface 14 is 69.984 degrees.

Moreover, a case where transparent glass is used as the material of light guide member 12 will now be discussed. The critical angle of light emitting to air from contact surface 14 of light guide member 12 is 41.472 degrees. On the other hand, the critical angle of light emitting from contact surface 14 of light guide member 12 to an affected part (e.g., hand) in contact with contact surface 14 is 67.995 degrees.

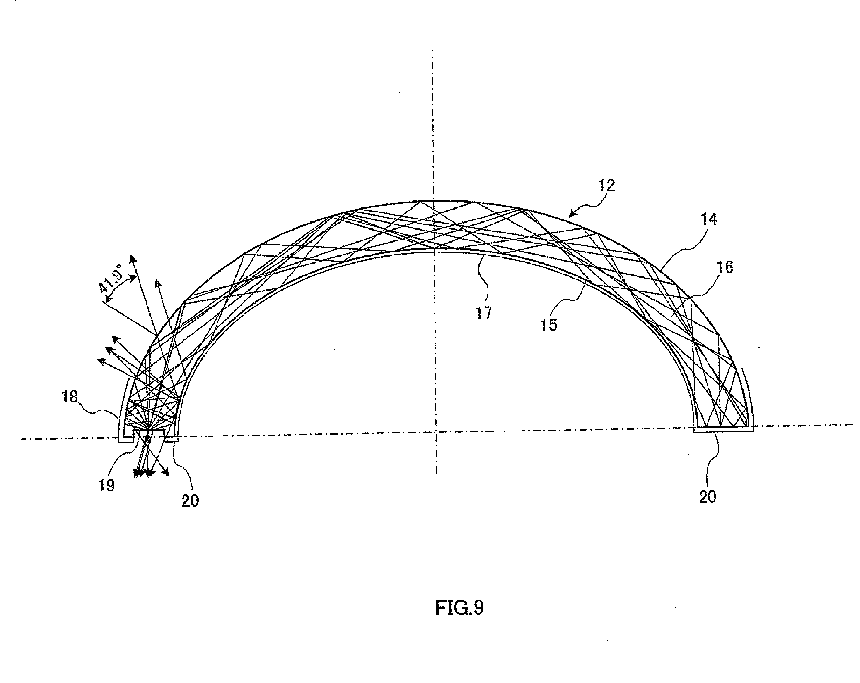

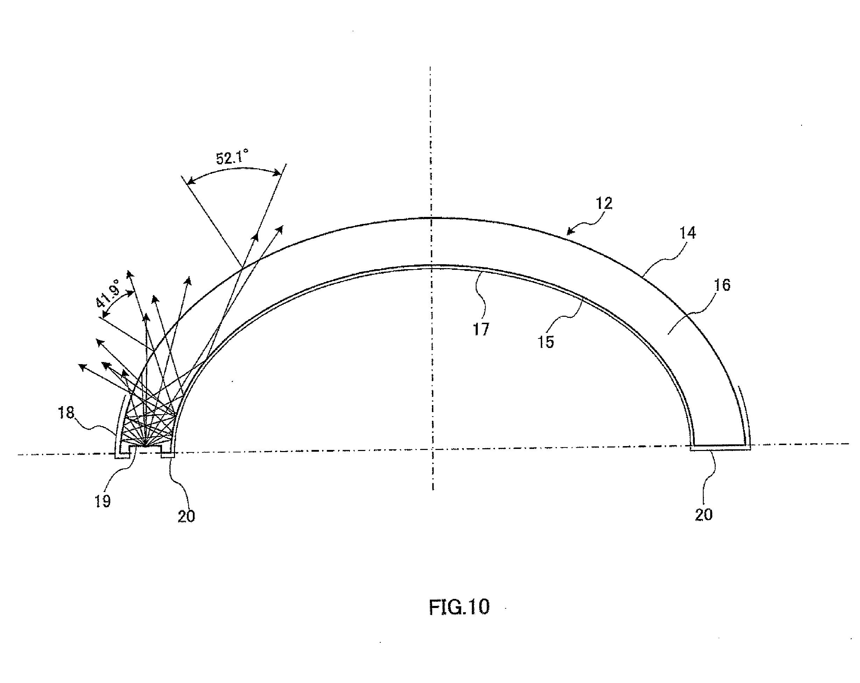

As described above, the critical angle of light emitting from contact surface 12 varies depending on whether or not the affected part is in contact with contact surface 14 of light guide member 12. Specifically, when the affected part is in contact with contact surface 14 of light guide member 12, the critical angle becomes large. The phototherapy device according to an embodiment of the present invention utilizes this difference in critical angle, thereby reducing the light emission from contact surface 14 not in contact with the affected part and allowing for selective emission of light from contact surface 14 in contact with the affected part. In this way, therapeutic efficiency is enhanced by selectively applying the light onto the affected part in contact with contact surface 14. The details of the mechanism by the therapeutic efficiency is enhanced will now be described with reference to FIG.9 and FIG.10.

As can be seen from FIG.3 and FIG.4, mounting body 13 has a linear shape extending in the direction perpendicular to the insertion direction from insertion port 8. Further, mounting body 13 has a semi-circular cross section. That is, mounting body 13 has a cross section of a so-called hog-backed shape. Further, a light source (second light source), which applies light onto mounting body 13, may also be provided. This allows the wrists to receive phototherapy.

Light emitted from each of the light sources respectively provided at a plurality of light incident ports 19 enters light guide path 16 through light incident port 19. As described above, it is preferable that the light source used in the phototherapy device according to an embodiment of the present invention emits light having a main wavelength range of near-infrared light. This is because near-infrared light has high body penetration.

The light sources provided at light incident ports 19 are provided such that their main optical axes are not opposite to each other. That is, the light sources are respectively provided so as to prevent the main optical axis of one of the light sources from passing the light incident port corresponding to the other one of the light sources. The term "main optical axis" as used herein means a line which connects points of maximum intensity of light, which light emits from the light source into light guide path 16.

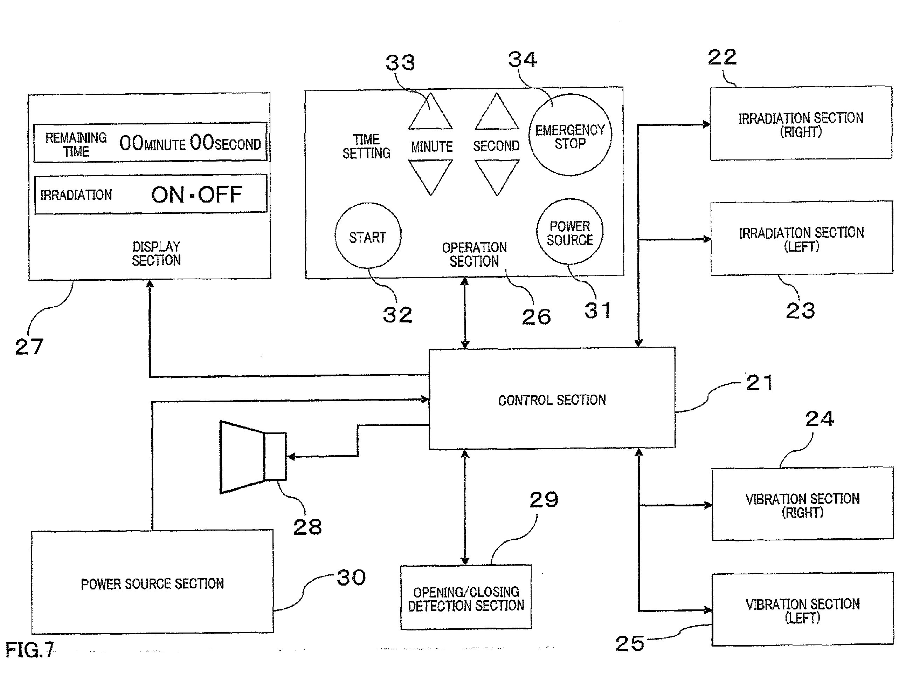

FIG.7 is a control block diagram of phototherapy device 2 of embodiment 1. Irradiation sections 22 and 23, vibration sections 24 and 25, operation section 26, display section 27, buzzer 28, and opening/closing detection section 29 are connected to control section 21, and electric power is supplied to each section from power source section 30.

Irradiation section 22 can supply electric power and a signal to the light source provided at right-side light guide member 12. Light irradiation section 23 can supply electric power and a signal to the light source provided at left-side light guide member 12.

Vibration section 24 can vibrate mounting body 13 provided on the insertion port 8 side of right-side light guide member 12. Vibration section 25 can vibrate mounting body 13 provided on insertion port side 8 of left-side light guide member 12.

As shown in FIG.4, operation section 26 is provided on the front side (insertion port 8 side) of the upper surface of lid 7, and includes power source switch 31, start switch 32, therapy time input switch 33, and emergency stop switch 34.

As shown in FIG.4, display section 27 is provided on the upper surface of lid 7 and can display the statement as to whether or not irradiation is underway, as well as the remaining time of irradiation. Buzzer 28 emits a warning sound, for example. Opening/closing detection section 29 detects the state where hook 10 is engaged with engagement hole 9 of base 5 and thereby lid 7 is closed.

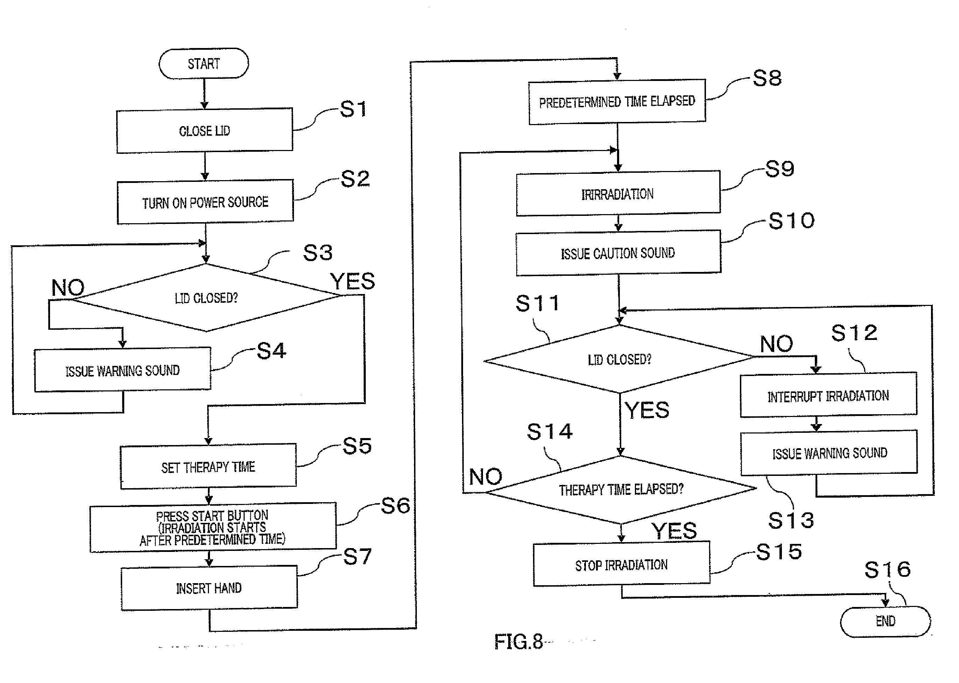

When phototherapy device 2 is operated with the above-described configuration, the procedure of the flow chart shown in FIG.8 is performed.

First, lid 7 is closed to cover the surface of base 5 and placed in the state shown in FIG.1 (S1 in FIG.8). Next, electric power is supplied by turning power source switch 31 on (S2 in FIG.8). Opening/closing detection section 29 then detects whether or not lid 7 is closed (S3 in FIG.8). Whether lid 7 is closed or not is determined by detecting whether or not hook 10 is engaged with engagement hole 9 of base 5.

When lid 7 is not closed at this time, buzzer 28 gives off an alarm sound (S4 in FIG.8). On the other hand, when lid 7 is closed, therapy time is set by using therapy time input switch 33 of operation section 26 (S5 in FIG.8).

When a signal is input by pressing start switch 32 of operation section 26 (S6 in FIG.8), two irradiation sections 22 and 23 are driven after a lapse of a predetermined standby time. That is, the light source is ready for applying light into light guide paths 16 of two light guide members 12.

As shown in FIG.2, patient 3 sitting on chair 4 inserts his/her hands via insertion ports 8 and bring the hands in contact with the contact surface of light guide members 12 (S7 in FIG.8), and waits for the predetermined standby time (S8 in FIG.8). Light from each of the light sources is allowed to enter light guide path 16 of each of left and right light guide members 12, and thereby irradiation is started (S9 in FIG.8).

During irradiation, left and right mounting bodies 13 may be vibrated by vibration sections 24 and 25 so as to provide vibration to body areas near the wrists of patient 3. Patient 3 can thereby receive a so-called massage.

During irradiation, buzzer 28 incessantly gives off a caution sound (e.g., beep-beep sound) (S10 in FIG.8). During irradiation opening/closing detection section 29 also detects whether or not lid 7 is closed (S11 in FIG.8). When it is determined that lid 7 is open, irradiation of light into light guide path 16 of light guide member 12 is stopped (S12 in FIG.8), and it is notified by buzzer 28 that lid 7 is open (S13 in FIG.8).

On the other hand, as long as it is detected by opening/closing detection section 29 that lid 7 is kept closed, irradiation is continued until the therapy time set by therapy time input switch 33 elapses (S14 in Fig.8). Then, when the therapy time has elapsed, irradiation of light into light guide member 12 is stopped (S15 in FIG.8), and the flow ends (S16 in FIG.8).

As described above, the phototherapy device according to an embodiment of the present invention utilizes changes in critical angle so as to prevent the emission of light from areas of contact surface 14 not in contact with an affected part, and to allow light to be selectively emitted from areas of contact surface 14 in contact with the affected part. This mechanism will now be described below.

Fig.9 shows a state where light from light sources propagates through light guide path 16 of light guide member 12. As shown in FIG.6, from each of a plurality of light sources provided at the lower end surface of light guide path 16 of light guide member 12 having the substantially semi-spherical shape, light is applied into light guide path 16. The light applied into light guide path 16 from each light source is first reflected by reflection member 18 of contact surface 14, which member is provided at the lower part of the spherical surface, or by reflection member 17 of opposite surface 15. Then, as shown in FIG.9, the light propagates while repeating reflection on opposite surface 15 and contact surface 14. Further, the light can propagate through light guide path 16 to reach, via the top part of light guide member 12 having the substantially semi-spherical shape, the opposite side lower part of light guide member 12.

Opposite surface 15 of light guide member 12 according to Embodiment 1 is total reflection surface 17. Therefore, the light propagating through light guide path 16 may be emitted only from contact surface 14. More specifically, among light beams which reach the inner surface of contact surface 14 of light guide member 12, only those that have an incident angle smaller than the critical angle are allowed to emit from contact surface 14.

Here, it is assumed that the material of light guide member 12 in FIG.9 is acrylic resin. The critical angle of light emitting from acrylic resin to air is 42.155 degrees. Therefore, only light beams which have reached the inner surface side of contact surface 14 at an incident angle smaller than 42.155 degrees emit from contact surface 14. That is, as shown in FIG.9, light beams which have reached the inner surface of contact surface 14 at an incident angle of 41.9 degrees, the angle being smaller than the critical angle (42.155 degrees), emit from contact surface 14.

Thus, light to be emitted from contact surface 14 needs to reach the inner surface of contact surface 14 at an incident angle smaller than the critical angle. Light which reaches the inner surface of contact surface 14 at an incident angle smaller than the critical angle in this way is mainly generated in the vicinity of light incident port 19 of the light source and in the vicinity of the lower end surface of light guide path 16. Therefore, when the reflection surface is arranged in the vicinity of light incident port 19 of the light source and in the vicinity of the lower end surface of light guide path 16 (when reflection members 18,20 are provided at these parts), the amount of light from contact surface 14 can be significantly reduced.

When reflection members 18,20 are provided, light entered into the light guide path from the light source is reflected by reflection members 18,20 so as to propagate back and forth in light guide path 16.

As a result of the foregoing, the amount of light emitted from contact surface 14 of light guide member 12 is significantly reduced in the state where air is in contact with (where an affected part is not in contact with) contact surface 14 of light guide member 12. Therefore, even when irradiation is performed for some reason in the state where lid 7 is open, it is possible to prevent intense light emitting from light guide member 12 from directly entering the eye of a patient.

On the other hand, it is now assumed that an affected part (for example, hand) is brought into contact with contact surface 14 of light guide member 12 formed of acrylic resin. The critical angle of light emitting from the acrylic resin to the affected part is 69.984 degrees. Therefore, light reaching the inner surface of contact surface 14 at an incident angle smaller than the critical angle (69.984 degrees) emits from contact surface 14. That is, as shown in FIG.10, even light which reaches the inner surface of contact surface 14 at an incident angle of 52.1 degrees, the angle being smaller than the critical angle (69.984 degrees), emits from contact surface 14.

As is apparent from the comparison between FIG.9 and FIG.10, it can be seen that, when an affected part (e.g., hand) is in into contact with contact surface 14, the amount of light emitting from contract surface 14 of light guide member 12 is increased.

As a result of the foregoing, when an affected part (e.g., hand) is in into contact with contact surface 14 of light guide member 12, the amount of light emitting from contact surface 14 of light guide member 12 is significantly increased. That is, light from the light source is selectively applied onto the affected part in contact with contact surface 14 of light guide member 12. Therefore, it is possible to enhance the therapeutic efficacy on the affected part while significantly reducing the amount of light emitting from areas of the contact surface of light guide member 12 that are not in into contact with the affected part.

Embodiment 1 is directed to an embodiment where light guide member 12 has a substantially semi-spherical shape, but the shape of light guide member 12 is not limited. That is, it is only necessary that the shape of light guide member 12 is formed such that light can be selectively applied onto an affected part (biological tissue) that is in contact with contact surface 14 of light guide member 12, and such that the amount of light emitting from areas of light guide member 12 that are not in contact with the affected part (biological tissue) can be significantly reduced. Examples of such shape of the light guide member include flat plate shape (see Embodiment 3).

### (Embodiment 2)

In Embodiment 2, a more preferable configuration of phototherapy device 2 of the present invention will be described.

As described above, symptoms of rheumatism usually appear in the joints. In particular, there are many joints in the area from the wrist to fingers of a hand, and hence symptoms of rheumatism usually appear there. Therefore, during phototherapy, it is necessary to intensively apply light in the area from the wrist to fingers of a hand.

Light may be applied to the entire affected part during phototherapy using phototherapy device 2, but intense light is required. For this reason, when it is intended to apply light onto an affected part entirely, a device configuration for applying high-power light is required, which results in large device size. When it is intended to apply intense light onto an affected part entirely, areas that are not in need of phototherapy are also irradiated. Irradiation onto such areas is undesirable from the viewpoint of energy efficiency. For this reason, in order to highly efficiently perform phototherapy using light from the light source, it may be preferable to selectively apply light onto an affected part in need of phototherapy.

In phototherapy device 2 according to Embodiment 2, the light source is provided so that the light power density of the part of contact surface 14 of light guide member 12, which part is in contact with an affected part in need of phototherapy, becomes relatively higher than the other part. Thereby, as described above,light can be selectively applied to the affected part in need of phototherapy.

That is, in phototherapy device 2 according to Embodiment 2, main optical axes 35 of light entered into light guide path 16 of light guide member 12 from a plurality of light sources are made to intersect each other in the light guide path of light guide member 12. Thereby, the light power density on contact surface 14 corresponding to the intersection part can be relatively increased as compared with the other part. The term "light power density" of a certain part means the intensity of light passing through the part in the light guide path. The affected part in contact with contact surface 14 with high light power density is expected to be irradiated with a large amount of light.

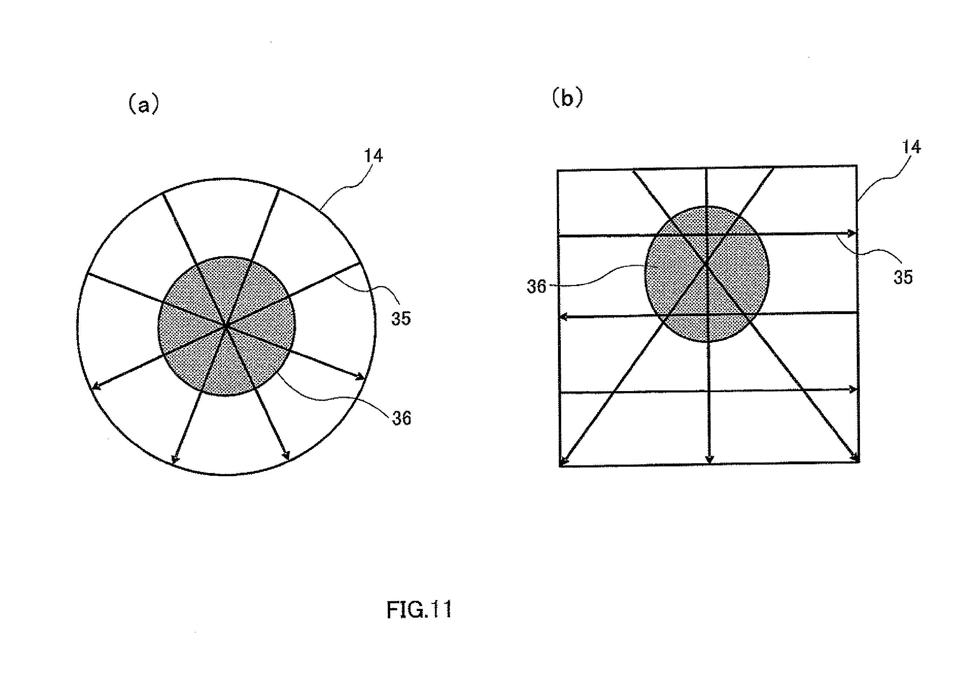

The light power density will be described in more detail with reference to FIG.11. FIG. 11 illustrates a state of main optical axes of light entered into guide path 16 from a plurality of light sources via the end surface of light guide member 12, as seen from the upper surface of light guide member 12. In light guide member 12, the light power density of the light entered into light guide member 12 from the plurality of light sources becomes relatively higher in the part where the optical axes of the plurality of light sources intersect each other than in the part where the optical axes of the plurality of light sources do not intersect each other. Therefore, by adjusting the arrangement of the light sources, a part of relatively high intersection density of the main optical axes and a part of relatively low intersection density of the main optical axes can be set in light guide member 12. That is, a part of high light power density and a part of low light power density can be arbitrarily set.

As shown in FIG.11(a) and FIG.11(b), affected part zone 36, which is an area with an affected part provided therein (brought into contact therewith), is set in contact surface 14 in phototherapy device 2 of the present invention. Further, the light sources are provided such that in light guide member 12 the number of the intersection of the main optical axes of light from the light sources is larger in affected part zone 36 than in areas other than affected part zone 36. This allows the light power density in affected part zone 36 to be relatively higher than the light power density in the area other than affected part zone 36.

The term "intersection of main optical axes" as used herein means that optical axes intersect each other in light guide path 16 which is defined between contact surface 14 and opposite surface 15, and that main optical axes intersect each other when the main optical axes.are observed in the thickness direction of the light guide member from the side of the contact surface (or from the opposite surface) of the light guide member. As described above, the term "intersection density of main optical axes" means the number of points at which two main optical axes intersect each other in a region of a predetermined area. When two main optical axes intersect each other, the number of the intersection points of the main optical axes is defined as one. When four main optical axes intersect each other at one point as shown in FIG.11(a), the number of intersection points of main optical axes is defined as six. This is because, when the four main optical axes are set as axis A, axis B, axis C, and axis D, there are six intersection points between axis A and axis B, between axis A and axis C, between axis A and axis D, between axis B and axis C, between axis B and axis D, and between axis C and axis D.

As shown in FIG.11(a), main optical axes 35 of light from a plurality of light sources may be made to intersect each other at one point. Also, as shown in FIG.11(b), main optical axes 35 of light from a plurality of light sources may be made to intersect each other at a plurality of points in affected part zone 36. How main optical axes 35 are made to intersect each other can be freely set according to the therapy.

Affected part zone 36, which is set in contact surface 14, can be freely set according to the size, shape and the like, of an affected part to be treated. By way of example, a case where a hand and fingers of a patient with rheumatism are treated will now be described. Rheumatism is a disorder that mainly afflicts adult men and women. For this reason, the size and shape of affected parts to be treated do not greatly vary from one individual to another. Therefore, when the phototherapy device is used to treat a hand and fingers of a patient with rheumatism, the shape and size of affected part zone 36 can be fixed. It is also possible to effectively perform therapy by concentrating the intersection points of the main optical axes on affected part zone 36.

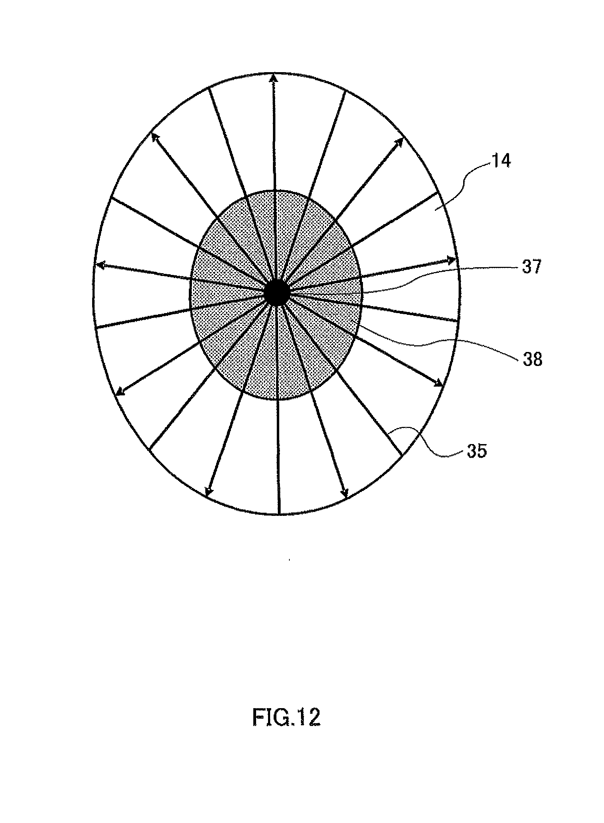

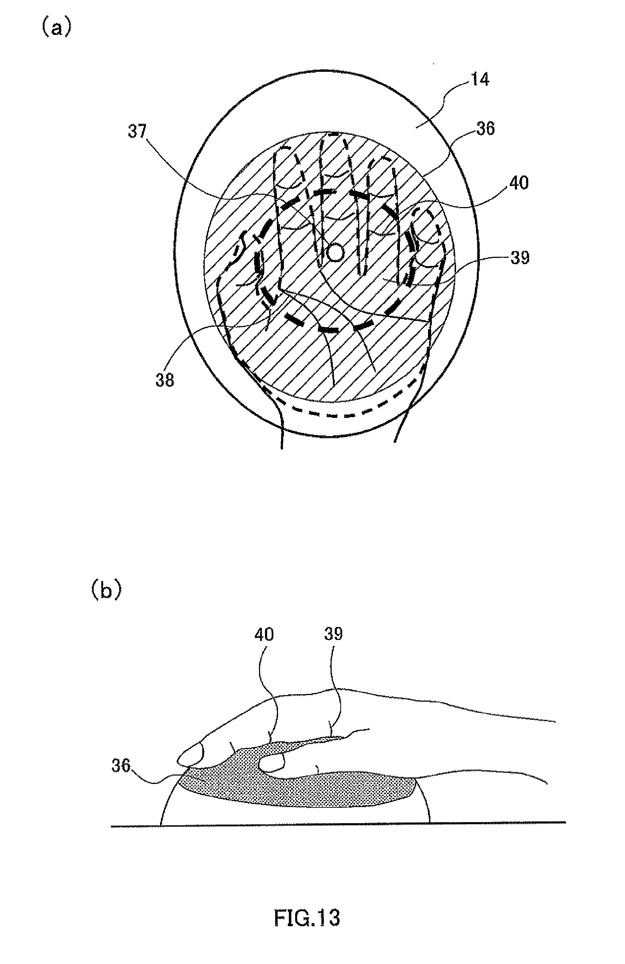

A method for setting affected part zone 36 will now be described with reference to FIG.12 and FIG.13. Similarly to Embodiment 1, FIG.12 and FIG.13 show an example in which affected part zone 36 is set in contact surface 14 of substantially semi-spherical light guide member 12.

FIG. 12 is a top view of contact surface 14 of substantially semi-spherical light guide member 12 (see FIG.5 and FIG.6). FIG.12 shows main optical axes 35 of light entered into light guide member 12 from a plurality of light sources. The plurality of light sources are respectively provided so that every main optical axis 35 of light entered into light guide path 16 of substantially semi-spherical light guide member 12 from the light sources crosses to a surrounding area of top part 37 of light guide member 12. That is, as shown in FIG.12, many intersection points of the main optical axes of light from the light sources exist in the surrounding area of top part 37. Therefore, the light power density in the surrounding area (top peripheral area 38) of top part 37 becomes highest in contact surface 14 of light guide member 12.

On the other hand, as shown in FIG.13(a) and FIG.13(b), an affected part (e.g., hand) of a patient is usually placed in top peripheral area 38 centering on top part 37 of light guide member 12. As can be seen from FIG.13(a) and FIG.13(b), the affected part is not necessarily placed on the entire surface of contact surface 14 of light guide member 12, but is usually provided in a predetermined area centering on top part 37 and corresponding to the size of the affected part.

Therefore, it is only necessary that affected part zone 36 be set in an area centering on top part 37 and that the size of affected part zone 36 be set according to the size of an affected part (e.g., hand). Thus, in the case where an affected hand is to be treated, it is preferable that affected part zone 36 be centered on top part 37 and top peripheral area 38, and sized enough to allow the area from metacarpophalangeal joints 39 to the vicinity of proximal finger joints 40 to be placed therein.

As described above, in Embodiment 2, the light power density is highest at top part 37 and in top peripheral area 38 of substantially semi-spherical light guide member 12. Therefore, phototherapy can be effectively performed in such a manner that, when a patient with rheumatism brings his/her hand (affected part) into contact with contact surface 14 of light guide member 12 in a comfortable posture, light is selectively and intensively applied to the area from metacarpophalangeal joints 39 to proximal finger joints 40, which is most in need of therapy.

### (Embodiment 3)

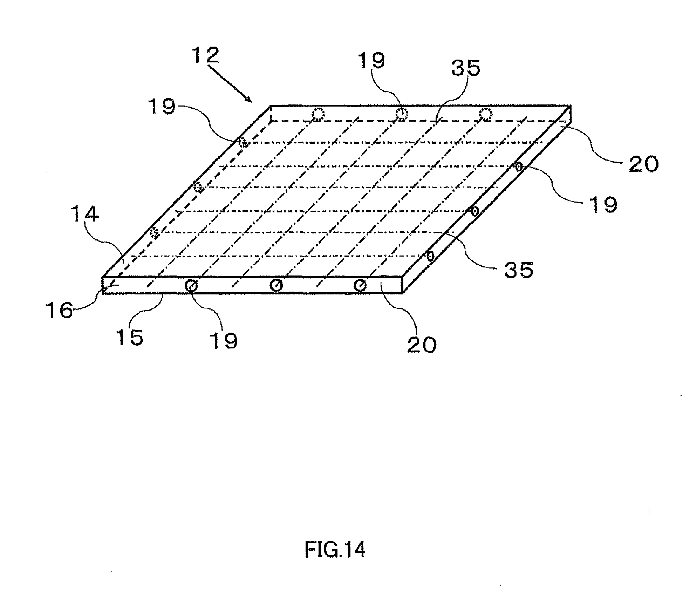

FIG.14 is a perspective view of a light guide member of a phototherapy device according to Embodiment 3 of the present invention.

In Embodiments 1 and 2, light guide member 12 has a substantially semi-spherical shape, and hence Embodiment 3 is different from Embodiments 1 and 2 in that light guide member 12 has a flat sheet shape. The other configuration according to Embodiment 3 is the same as that according to Embodiments 1 and 2. Therefore, in Embodiment 3, only light guide member 12 is described, and the description and illustration of the other configuration are omitted.

As shown in FIG.14, light guide member 12 has a substantially flat sheet shape. Similarly to Embodiment 1, light guide member 12 is made of material having a refractive index equal to or higher than a refractive index of a biological tissue, such as acrylic resin or transparent glass.

One of the sheet surfaces of sheet-like light guide member 12 is contact surface 14 to be contacted with an affected part (biological tissue), and the other surface is opposite surface 15 which opposes contact surface 14. Light guide path 16 is defined between contact surface 14 and opposite surface 15.

A reflection member (not shown in FIG.14) for reflecting light to contact surface 14 is provided on the entire surface of opposite surface 15. The reflection member is, for example, a reflection film. A plurality of light incident ports 19 are provided in the side surface of light guide member 12, and reflection surface 20 for reflecting light to light guide path 16 is provided on the side surface of light guide member 12 except light incident ports 19.

A light source is provided at each of the plurality of light incident ports 19 (not shown in Fig.14). The light sources are provided such that their main optical axes 35 are not opposite to each other (see FIG.11). Light beams from the light sources provided at light incident ports 19 enter light guide path 16 and spread out at a predetermined divergence angle. The light sources are provided such that main optical axes 35, along each of which the intensity of light beams spreading out at a predetermined divergence angle is highest, are not opposite to each other. With this configuration, the light intensity distribution in the light guide path of light guide member 12 can be uniform. It is also possible to reduce an amount of light entered into the light source provided at one of light incident ports 19, among a light emitted from a light source provided at the other of light incident ports 19.

As described in Embodiment 2, another preferable mode is that affected part zone 36 is set in contact surface 14 and the light sources are provided so that the light power density in affected part zone 36 is relatively high. On the other hand, in the case where contact surface 14 is a flat surface as in light guide member 12 having a substantially flat sheet shape in Embodiment 3, when light is uniformly applied onto the entire area of contact surface 14, light can be easily applied onto the entire area of the affected part.

In light guide member 12 in Embodiment 3, light entered into light guide path 16 through light incident port 19 propagates through light guide path 16 while being reflected on contact surface 14 and opposite surface 15. Similarly to Embodiment 1, light emission from the part of contact surface 14, which part is not in contact with the affected part, is significantly suppressed. On the other hand, light is selectively emitted from the part of contact surface 14, which part is in contact with the affected part. Thereby, light is applied onto the affected part so that the affected part receives phototherapy.

Also, with light guide member 12 of Embodiment 3, the same effect as that in Embodiment 1 can be obtained by configuring phototherapy device 2 similar to phototherapy device 2 according to Embodiment 1 and by similarly operating and using phototherapy device 2.

The light guide member in the phototherapy device shown in Embodiments 1 to 3 has a substantially semi-spherical shape or a substantially flat sheet shape, but the shape of the light guide member is not limited in particular. For example, the light guide member may be formed in a substantially disk-shaped sheet, belt-shaped sheet or other shape. The mode in which an affected part is brought into contact with the light guide member is not particularly limited; an affected part may also be brought into contact with the light guide member by winding the light guide member around the affected part.

That is, it is only necessary that the light guide member be made of material having a refractive index equal to or higher than a refractive index of a biological tissue. And also, the light guide member is provided with a contact surface and an opposite surface so as to allow light to be entered into the light guide path between the contact surface and the opposite surface.

### (Embodiment 4)

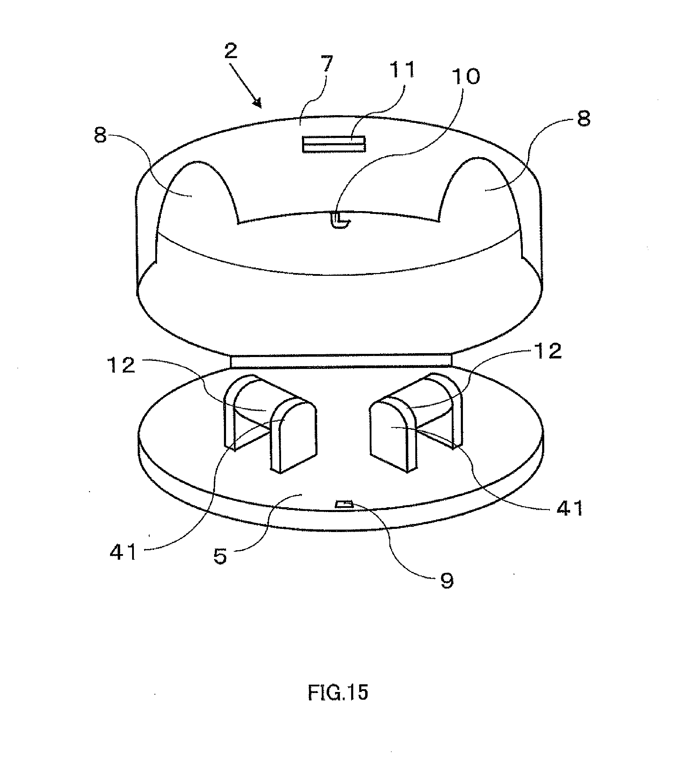

Phototherapy device 2 shown in FIG.15 has two light guide members 12 provided at a predetermined spacing on the surface of base 5. Light guide member 12 has a substantially rod shape. Each of two light guide members 12 is arranged above the surface of base 5 via holding sections 41 connected to the both ends of each of two light guide members 12.

The material of light guide member 12 is the same as in Embodiment 1, and needs only to have a refractive index equal to or higher than a refractive index (approximately 1.4) of a biological tissue (e.g., skin).

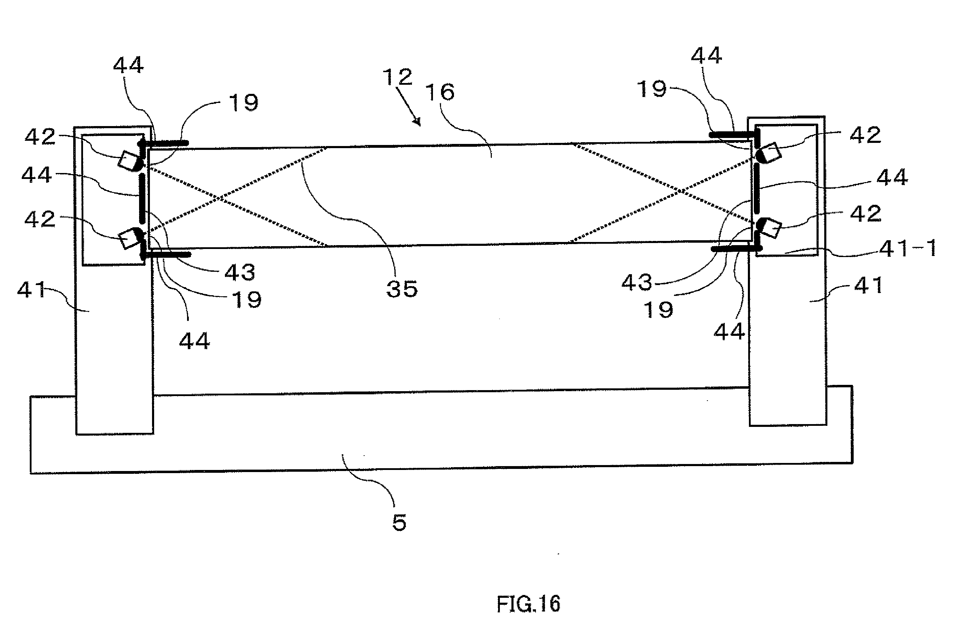

Further, as shown in FIG.16, light sources 42 are installed at both ends of light guide member 12. Light sources 42 are covered by holding section 41. That is, light guide member 12 configures light guide path 16. In FIG.16, light sources 42 are provided at both ends of light guide member 12, but may also be provided only at one end of light guide member 12.

Main optical axis 35 of light source 42 is directed to the inner side surface of light guide member 12. The light emitted from light source 42 enters light guide path 16 through light incident port 19 provided in end surface 43 of light guide member 12.

Furthermore, reflection member 44, which reflects light to the inside of light guide path 16, is provided also in a part of end surface 43 of light guide member 12 except light incident port 19. Further, reflection member 44, which reflects light to the inside of light guide path 16, is also provided at the joining part between light guide member 12 and holding section 41, and at the end part of the side surface of light guide member 12.

It is preferable that inner surface 41-1 of holding section 41 is a surface for reflecting or shielding the light which is emitted from light source 42 and which leaks without entering light guide path 16. It is thus possible to prevent leakage of light from holding section 41 to the outside.

In the configuration shown in Embodiment 4, when patient 3 grasps light guide member 12, patient 3 can bring an affected part (hand) into contact with the side surface of light guide member 12, so as to receive phototherapy of the affected part. Of course, even when patient 3 does not grasp light guide member 12 but simply brings an affected part into contact with light guide member 12, patient 3 can also receive phototherapy for the affected part.

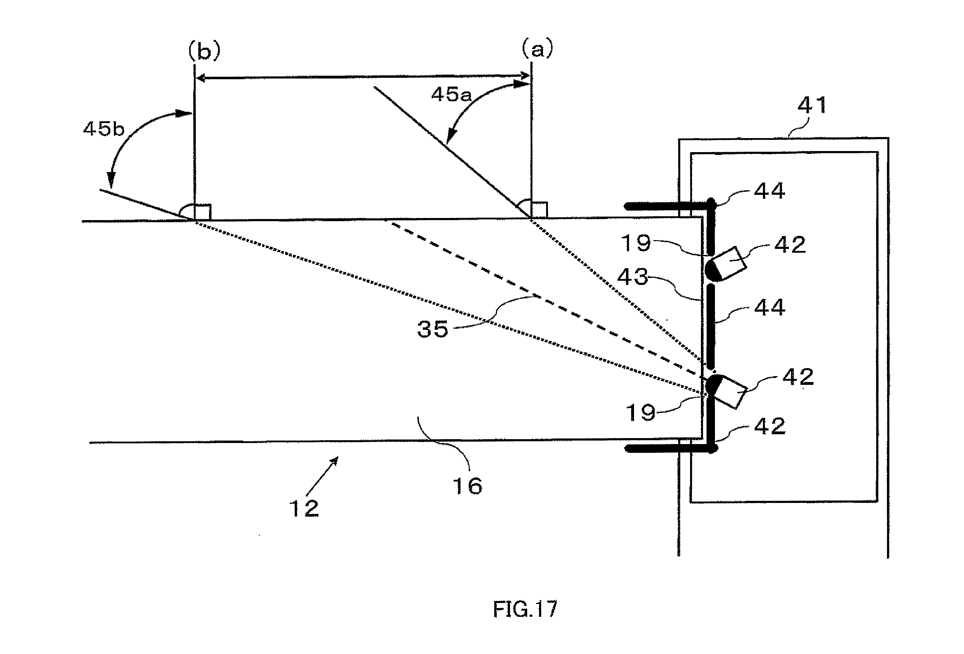

FIG.17 shows one end of light guide member 12 having a cylindrical shape, and shows a state where light from light source 42 enters light guide path 16 of light guide member 12. It is assumed that the material of light guide member 12 is acrylic resign.

An embodiment where an affected part (biological tissue) is not in contact with light guide member 12 will now be described. Light emitted from light source 42 enters light guide path 16 of light guide member 12. Critical angle 45a of light emitted to the air (refractive index: 1.00) from light guide member 12 made of acrylic resin (refractive index: 1.49) is 42.155 degrees. For this reason, when light entered into light guide path 16 reaches the inner side surface of light guide member 12 at an incident angle larger than critical angle 45a, the light is totally reflected on the inner side surface of light guide member 12. Further, the totally reflected light propagates through light guide path 16 while repeating reflection on the inner side surface of light guide member 12.

On the other hand, when light entered into light guide path 16 reaches the inner side surface of light guide member 12 at an incident angle smaller than critical angle 45a, the light is emitted to the outside from light guide path 16 of light guide member 12.

On the other hand, an embodiment where an affected part (biological tissue) is in contact with light guide member 12 will now be described. Critical angle 45b of light emitted from acrylic resin (refractive index: 1.49) to skin (refractive index: 1.4) of a biological tissue (affected part) is 69.984 degrees. Critical angle 45b is larger than critical angle 45a of light emitted to air (refractive index: 1.00) from acrylic resin (refractive index: 1.49). That is, the requirement for light reflection on the part of light guide member 12 is changed, which part is in contact with the affected part.

In this way, among light entering light guide path 16 of light guide member 12, light reaching the inner side surface of light guide member 12 at an incident angle larger than critical angle 45b is not emitted to the outside from light guide path 16 of light guide member 12. On the other hand, among the light entering light guide path 16 of light guide member 12, light reaching the inner side surface of light guide member 12 at an incident angle smaller than critical angle 45b is emitted from light guide path 16 of light guide member 12 to the affected part which is brought into contact with light guide member 12.

That is, when a biological tissue or affected part is not in contact with the area between (a) and (b) of light guide member 12 in FIG.17, light emits to the outside from light guide path 16. However, when the biological tissue is brought into contact with the area between (a) and (b) of light guide member 12, light emits from the part in contact with the biological tissue so as to enter the affected part.

In this way, light is selectively emitted to the affected part in contact with light guide member 12. As a result, much light is applied onto the affected part in contact with light guide member 12, and light emission from the part not in contact with the affected part is significantly reduced. Phototherapy can thus be effectively performed with intense light for phototherapy, and the intense light can be prevented from directly being applied to the eye.

In order to allow the selective emission of light from the part of light guide path 16 of light guide member 12, which part is in contact with the affected part, it is only necessary that the refractive index of the material of light guide member 12 be set to be equal to or higher than the refractive index (approximately 1.4) of the affected part (e.g., the skin of a biological tissue). Thereby, the effect of selectively applying light onto the affected part can be sufficiently achieved.

In the case where light guide member 12 is made of acrylic resin, it is preferable to provide light source 42 such that the incident angle of main optical axis 35 of light from light source 42 with respect to the inner side surface of light guide member 12 is set to an angle of 42.155 degrees (critical angle 45a) or more to 69.989 degrees (critical angle 45b) or less.

That is, when the incident angle of main optical axis 35 of light from light source 42 with respect to the inner side surface of light guide member 12 is set to an angle of 42.155 degrees (critical angle 45a) or more to 69.989 degrees (critical angle 45b) or less, light emission from the part of light guide member 12, which part is not in contact with the affected part, can be effectively reduced, and light emission from the part of light guide member 12, which part is in contact with the affected part, can be effectively promoted. Therefore, intense light to for the phototherapy can be prevented from directly entering the eye, and hence phototherapy can be performed more safely.

Reflection member 44 may be provided on the side surface of light guide member 12 except the part of the side surface of light guide member 12, which part is brought into contact with the affected part. Thereby, light entered into light guide member 12 from light source 42 can be more efficiently applied onto the affected part, so as to enhance the therapeutic efficacy. For example, reflection member 44 can be provided from the end part to (a) of the side surface of light guide member 12 in FIG.17. Thereby, the amount of light applied from a part of the side surface of light guide member 12 is reduced, which part is other than the part in contact with the affected part, so that the light can be selectively applied onto the affected part. As a result, efficient therapy of the affected part can be achieved.

In Embodiment 4, light guide member 12 has a cylindrical shape, but the shape of light guide member 12 is not limited in particular. For example, the light guide member may have a substantially rod shape which is bent partially or entirely, or a substantially rod shape in which the cross section of the light guide path is formed in a shape other than circular, such as oval or polygonal shape. Phototherapy device 2 according to Embodiment 4 has two light guide members 12 (see FIG.15), but two light guide members 12 may be connected to each other so as to be integrated with each other.

In Embodiment 4, the main optical axis of light from each of light sources 42 directly reaches the inner side surface of the part of light guide member 12, which part is brought into contact with an affected part, but this is not necessarily needed. For example, each of light sources 42 may be provided so that the main optical axis of light from each of light sources 42 is first reflected on reflection surface 44 of the side surface of light guide member 12, so as to allow the reflected light to reach the part of light guide member 12, which part is brought into contact with the affected part. Light guide member 12 of Embodiment 4 has a rod shape, and the thickness of light guide member 12 is substantially uniform. For this reason, light propagating through light guide member 12 undergo repetitive reflections on the inner side surface of light guide member 12, but the reflection angle is almost constant. Therefore, even when the main optical axis of light from each of light sources 42 does not directly reach the part in contact with the affected part but reaches the part in contact with the affected part after being reflected on the inner side surface of light guide member 12, the same effect can be obtained.

### (Embodiment 5)

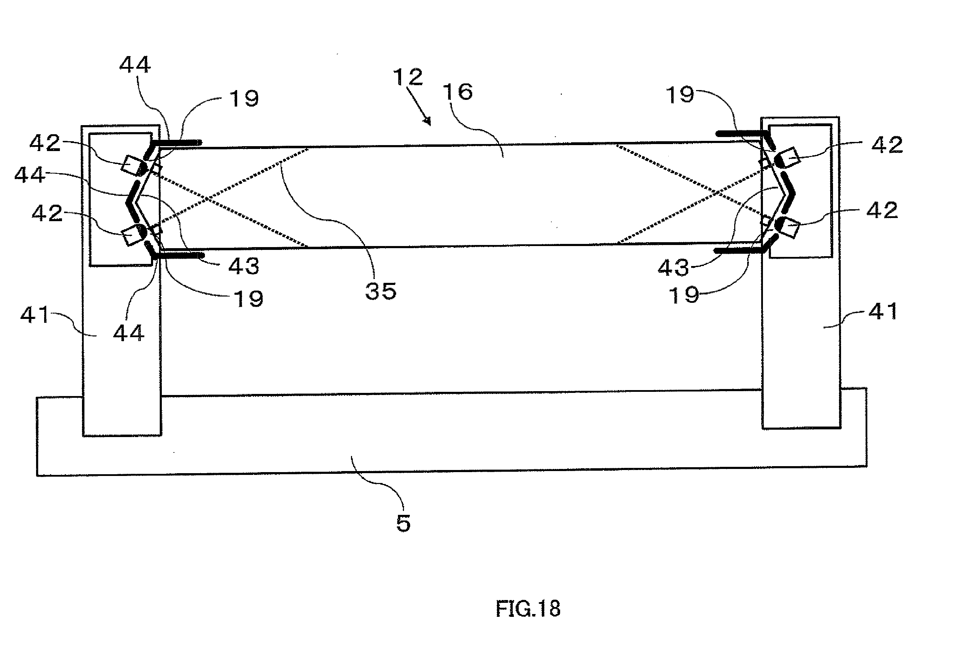

FIG.18 is a cross-sectional view of light guide member 12 of a phototherapy device according to Embodiment 5. End surface 43 of cylindrical light guide member 12 of the phototherapy device according to Embodiment 4 has a plane shape, while end surface 43 of light guide member 12 of the phototherapy device according to Embodiment 5 has a multi-sided pyramid shape. The other configuration in Embodiment 5 is the same as the configuration in Embodiment 4.

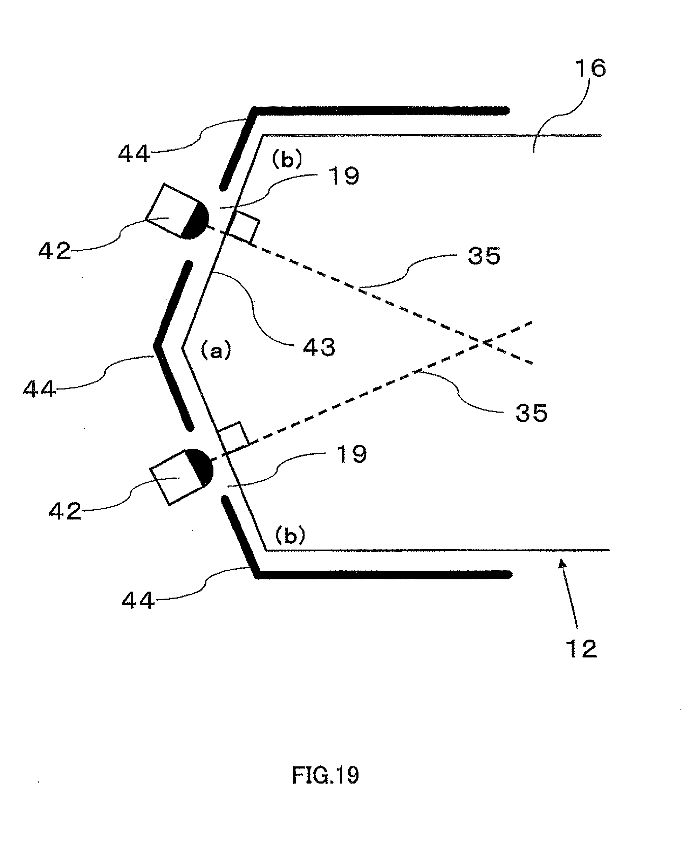

As shown in FIG.18, end surface 43 of light guide member 12 in Embodiment 5 is formed in a multi-sided pyramid shape. For this reason, when light emitted from light source 42 enters light guide path 16 of light guide member 12, main optical axis 35 of the light perpendicularly intersects one surface of the multi-sided pyramid body (see FIG.19).

On the other hand, as shown in FIG.17 (Embodiment 4), in the case where end surface 43 of light guide member 12 is a plane perpendicular to the side surface of light guide member 12, when light emitted and diffused from light source 42 enters light guide path 16 of light guide member 12, a part of the light is reflected on end surface 43 and cannot enter light guide path 16. For this reason, some of the light beam from light source 42 cannot be used for phototherapy. That is, as the incident angle of main optical axis 35 of light emitted and diffused from light source 42 with respect to end surface 43 is deviated from 90 degrees, more light may be reflected on end surface 43.

On the other hand, when the end surface 43 is formed in a multi-sided pyramid shape (see Fig.18 and Fig.19), main optical axis of light emitted from light source 42 can be substantially perpendicular to end surface 43. As a result, the reflection of light by end surface 43 can be suppressed, and the phototherapy with higher energy efficiency can be achieved.

End surface 43 of light guide member 12 of the phototherapy device according to Embodiment 5 has a multi-sided pyramid shape, but the shape of end surface 43 of light guide member 12 is not limited in particular. The shape of end surface 43 of light guide member 12 may be, for example, a cone shape and the like. In short, as long as end surface 43 of light guide member 12 is configured to allow the main optical axis of light from light source 42 to be substantially perpendicular to light incident port 19, the shape of end surface 43 of light guide member 12 is not limited in particular and can be suitably set according to the shape of light guide member 12.

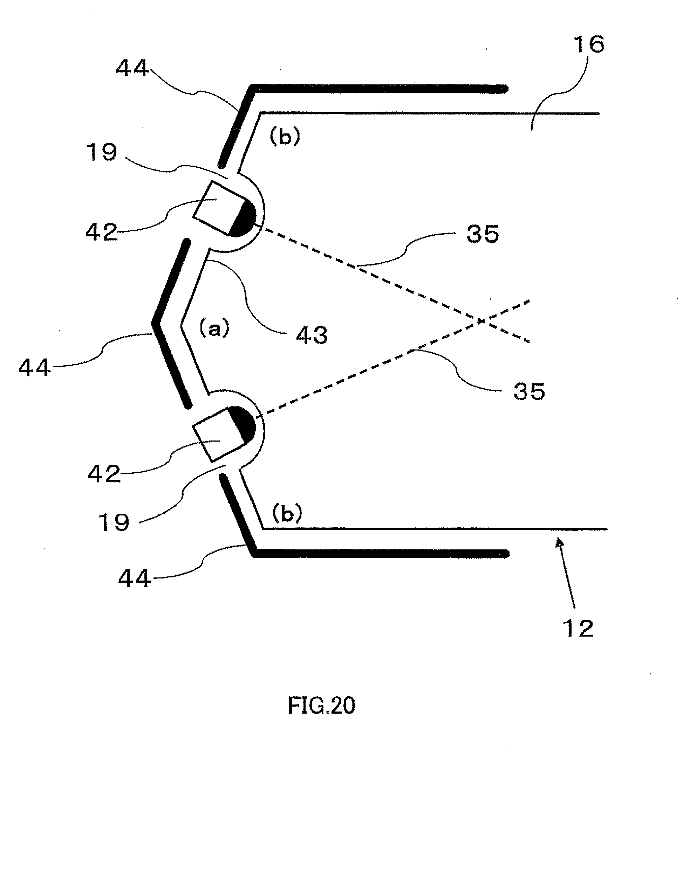

Further, as shown in FIG.20, end surface 43 of light guide member 12 of the phototherapy device may also be configured in such a manner that depressions are provided in end surface 43 of light guide member 12, and that each of the depressions is used as light incident port 19 of light from light source 42. Light source 42 is provided so that the light emitted and diffused from light source 42 directs perpendicularly to the wall surface of the depression. For this reason, it is preferable that light source 42 be provided in the inside of the depression. Thereby, a larger amount of light from light source 42 can enter light guide path 16. That is, with the configuration shown in FIG.20, the light emitted from light source 42 can be significantly suppressed from being reflected on end surface 43 and light incident port 19, and hence the light can most efficiently enter light guide path 16 of light guide member 12.

### Industrial Applicability

As described above, with the phototherapy device of the present invention, light can be selectively and intensively applied onto an affected part to be treated. That is, the phototherapy device of the present invention is configured such that, when an affected part (e.g., hand) is brought into contact with the contact surface of the light guide member of the phototherapy device, intense light is selectively emitted from the part in contact with the affected part so as to be applied onto the affected part. Thereby, a finite light of the light source can be efficiently used for phototherapy.

The conventional phototherapy devices require larger device size for radiation of high-power light or, where upsizing is not intended, need to reduce the radiation area. By contrast, the phototherapy device of the present invention eliminates the need to increase the device size and to reduce the irradiation area. It is also possible to prevent light from being applied onto unwanted areas other than the affected part. This leads to downsizing, increased therapeutic efficiency, and improved energy efficiency of the phototherapy device.

With the phototherapy device of the present invention, when an affected part (e.g., hand) is brought into contact with the contact surface of the light guide member, intense light is emitted only from the part of the contact surface, which part is in contact with the affected part, and is applied onto the affected part. That is, it is possible to reduce light emission from a part of the contact surface, which part is not in contact with the affected part. As a result, it is possible to prevent direct entry of intense light into the eye.

With the above-described configuration, intense light from a first light source can be selectively applied onto an affected part in contact with the contact surface of the light guide member. Therefore, it is not required to adjust an irradiated area to the shape and size of the affected part and to follow the movement of the affected part. As a result, it is not necessary to use a complicated device that performs irradiation while following the movement of the affected part.

The phototherapy device of the present invention is therefore expected to be widely used as a phototherapy device for alleviating intense pain due to chronic non-infectious inflammation in muscles and joints, as well as for treating rheumatoid arthritis. Also, as for affected parts to receive phototherapy, the phototherapy device of the present invention can be extensively used for the hands, feet and other body parts.

The present application claims priority to Patent Application No. JP2010-028474 filed in Japan on February 12, 2010, and Patent Application No. JP2010-055745 filed in Japan on March 12, 2010, the entire contents of which are hereby incorporated by reference.

### Reference Signs List

- 1: Desk
- 2: Phototherapy device
- 3: Patient
- 4: Chair
- 5: Base
- 6: Hinge
- 7: Lid
- 8: Insertion port
- 9: Engagement hole
- 10: Hook
- 11: Hook button
- 12: Light guide member
- 13: Mounting body
- 14: Contact surface
- 15: Opposite surface
- 16: Light guide path
- 17, 18, 20: Reflection member
- 19: Incident port
- 21: Control section
- 22, 23: Irradiation section
- 24, 25: Vibration section
- 26: Operation section
- 27: Display section
- 28: Buzzer
- 29: Opening/closing detection section
- 30: Power source section
- 31: Power source switch
- 32: Start switch
- 33: Therapy time input switch
- 34: Emergency stop switch
- 35: Main optical axis
- 36: Affected part zone
- 37: Top part
- 38: Top peripheral area
- 39: Metacarpophalangeal joint
- 40: Proximal finger joint
- 41: Holding section
- 41-1: Inner surface of holding section
- 42: Light source
- 43: End surface
- 44: Reflection member
- 45a, 45b: Critical angle

## Claims

1. A phototherapy device comprising:
a light guide member including a contact surface to be contacted with an affected part, and an opposite surface opposing the contact surface, the light guide member serving as a light guide path; and
one or more first light sources for applying light having a main wavelength in a range of near-infrared light into the light guide member,
wherein a material constituting the light guide member has a refractive index equal to or higher than a refractive index of a biological tissue.

2. The phototherapy device according to claim 1, wherein
the material constituting the light guide member has a refractive index of 1.4 or more.

3. The phototherapy device according to claim 2, wherein
the material constituting the light guide member is one of light-transmissive acrylic resin, light-transmissive polyethylene terephthalate resin, light-transmissive silicone rubber, and light-transmissive glass.

4. The phototherapy device according to any one of claims 1 to 3, wherein
the light guide member includes a light guide path end surface provided with one or more light incident ports configured to receive light from the first light sources, and
two or more of the first light sources are arranged at the light guide path end surface.

5. The phototherapy device according to claim 4, wherein
the light guide path end surface includes two or more of the light incident ports respectively corresponding to the first light sources.

6. The phototherapy device according to claim 4 or 5, wherein
the first light sources are provided such that main optical axes of the first light sources are not opposite to each other.

7. The phototherapy device according to any one of claims 4 to 6, wherein
the first light sources are provided such that the main optical axis of one of the first light sources and the main optical axis of another of the first light sources intersect each other in the light guide path of the light guide member.

8. The phototherapy device according to claim 7, wherein
the contact surface includes an affected part zone arbitrarily set according to the affected part, and
the affected part is brought into contact with the contact surface in the affected part zone.

9. The phototherapy device according to claim 8, wherein
the first light sources are provided such that a density of intersections of the main optical axes of the first light sources is higher in the affected part zone than outside the affected part zone.

10. The phototherapy device according to any one of claims 1 to 9, further comprising a reflection member provided on the opposite surface and for reflecting light to a contact surface side.

11. The phototherapy device according to any one of claims 4 to 9, further comprising a reflection member for reflecting light to the opposite surface, the reflection member provided on the end surface of the light guide path and on the contact surface near the end surface of the light guide path.

12. The phototherapy device according to any one of claims 4 to 9, further comprising a reflection member for reflecting light to the inside of the light guide path, the reflection member provided on the end surface of the light guide path except the light incident port of the first light source.

13. The phototherapy device according to any one of claims 1 to claim 12, wherein
the light guide member forms a hollow semi-spherical body, and
the contact surface is the outer surface of the hollow semi-spherical body, and the opposite surface is the inner surface of the hollow semi-spherical body.

14. The phototherapy device according to claim 13, comprising
two or more of the first light sources,
wherein each of the first light sources is provided such that the main optical axis of one of the first light sources and the main optical axis of another of the first light sources intersect each other at a top part or in the vicinity of the top part of the light guide member forming the hollow semi-spherical body.

15. The phototherapy device according to any one of claims 1 to 14, further comprising a base on which the light guide member is arranged.

16. The phototherapy device according to claim 15, further comprising
a lid covering, in an openable and closable manner, a surface of the base on which the light guide member is arranged,
wherein an insertion port for inserting the affected part to be brought into contact with the contact surface of the light guide member is provided at either one or both of the lid and the base.

17. The phototherapy device according to claim 16, further comprising a mounting body provided on the surface of the base at a position between the insertion port and the light guide member.

18. The phototherapy device according to claim 17, further comprising a second light source for supplying light to the mounting body.

19. The phototherapy device according to one claim 17 or 18, further comprising a vibration unit connected to the mounting body.

20. The phototherapy device according to any one of claim 1 to claim 19, wherein the phototherapy device is used to treat a hand, foot or finger of a patient with rheumatism.
